# EUROPÄISCHE PATENTSCHRIFT

(11) **EP 1 909 601 B1**
(45) Veröffentlichungstag und Bekanntmachung des Hinweises auf die Patenterteilung: **22.05.2013**
(21) Anmeldenummer: 06776542.0
(22) Anmeldetag: 01.08.2006
(51) Int. Cl.: A23L 1/305, A23L 1/302, A61K 31/198, A61K 31/205, A61K 31/155, A23L 2/52, A23C 9/152, A23C 9/158, A23C 21/08

(54) **FLÜSSIG-FORMULIERUNG AUF BASIS EINER GUANIDINOESSIGSÄURE-KOMPONENTE**
LIQUID FORMULATION BASED ON A GUANIDINOACETIC ACID COMPONENT
FORMULATION LIQUIDE A BASE D'UN CONSTITUANT ACIDE GUANIDINOACETIQUE

(30) Priorität: 02.08.2005 DE 102005036244
(43) Veröffentlichungstag der Anmeldung: 16.04.2008
(73) Patentinhaber: AlzChem Trostberg GmbH, 83308 Trostberg (DE)
(72) Erfinder: GASTNER, Thomas, 84549 Engelsberg (DE); KRIMMER, Hans-Peter, 84558 Kirchweidach (DE); STURM, Werner, 83342 Tacherting (DE)
(74) Vertreter: Dey, Michael
(86) Internationale Anmeldenummer: PCT/EP2006/007609
(87) Internationale Veröffentlichungsnummer: WO 2007/014756

(56) Entgegenhaltungen:
- WO-A-91/07954
- WO-A-2004/000297
- WO-A-2005/120246
- WO-A-2006/092298
- WO-A2-2004/000042
- US-A- 2 761 807

## Beschreibung

Gegenstand der vorliegenden Erfindung ist eine neue Zubereitung für die menschliche Ernährung, die als ernährungsphysiologisch wirksamen Bestandteil eine Guanidinoessigsäure-Komponente und einen Methylgruppendonor aus der Reihe Cholin, Methionin oder Betain enthält.

Guanidinoessigsäure wurde erstmals von C. J. Weber im Jahre 1,934 aus dem Urin von Hunden und Menschen isoliert. Bereits Weber vermutete, dass es sich um den metabolischen Vorläufer von Kreatin handelt (Weber, C. J., Proc. Sot. Exp. Biol. and Med., 33, 172 (1934)).

Wenig später konnte gezeigt werden, dass Guanidinoessigsäure tatsächlich eine bei Tieren und auch im Menschen vorkommende körpereigene Substanz ist, welche bei der Biosynthese des Kreatins eine zentrale Rolle einnimmt. Kreatin kann sowohl durch die Nahrung aufgenommen als auch endogen gebildet werden. Die Kreatin-Biosynthese geht von Glycin und L-Arginin aus. Bei Säugetieren wird vor allem in den Nieren, aber auch in der Leber und der Bauchspeicheldrüse, durch das Enzym Aminotransferase die GuanidinoGruppe des L-Arginins gespalten und eine N-C-N-Gruppe auf das Glycin übertragen. Das L-Arginin wird hierbei in L-Ornithin umgewandelt. Die so gebildete Guanidinoessigsäure wird im nächsten Schritt, bei Vertebraten geschieht dies ausschließlich in der Leber, mit Hilfe des Enzyms Transmethylase in Kreatin umgewandelt. Hierbei dient S-Adenosylmethionin als Methylgruppen-Donor. Das Kreatin diffundiert anschließend in den Blutkreislauf und wird so zu den Zielorganen transportiert. Der Transport durch die Zellmembran in die Zellen geschieht hierbei durch einen spezifischen Kreatin-Transporter.

Kreatin nimmt im Energiestoffwechsel der Zelle eine wichtige Rolle ein, wobei es als energiereiches Phosphokreatin neben dem Adenosintriphosphat (ATP) eine wesentliche Energiereserve des Muskels darstellt. Im Ruhezustand des Muskels kann ATP auf Kreatin eine Phosphat-Gruppe übertragen, wobei Phosphokreatin gebildet wird, welches dann im direkten Gleichgewicht mit ATP steht. Bei Muskelarbeit ist es von entscheidender Bedeutung, die ATP-Vorräte schnellstmöglich wieder aufzufüllen. Hierfür steht in den ersten Sekunden maximaler Muskelbelastung das Phosphokreatin zur Verfügung. Hierbei kann in einer sehr schnellen Reaktion durch das Enzym Kreatinkinase eine Phosphatgruppe auf Adenosindiphosphat übertragen und somit ATP zurückgebildet werden. Dies wird auch als Lohmann-Reaktion bezeichnet.

Kreatin ist seit langem als geeignetes Nahungsergänzungs- und Futtermittel bekannt. Bei starker und über längere Zeit anhaltender Muskelarbeit sind die natürlich im Körper vorhandenen Kreatinvorräte rasch erschöpft. Aus diesem Grund haben sich insbesondere bei Leistungssportlern gezielte Kreatingaben positiv auf die Ausdauer und Leistungsfähigkeit ausgewirkt, wobei unerwünschte Anreicherungsprozesse im Körper oder nachteilige Abbauprodukte unbekannt sind. Der Grund hierfür ist darin zu sehen, dass Kreatin bei einer übermäßigen Zufuhr vom Körper über die Nieren ausgeschieden wird. Weiterhin wird Kreatin mit einer konstanten Rate in das zyklische Abfallprodukt Kreatinin umgewandelt, welches ebenfalls über die Nieren ausgeschieden wird. Dies stellt somit einen zweiten metabolischen Abbauweg dar.

Weiterhin ist bekannt, dass eine Kreatin-Supplementierung zu einer Erhöhung der Körpermasse führt. Dies ist zu Anfang auf eine vermehrte Aufnahme von Wasser in den Muskel zurückzuführen. Langfristig führt das Kreatin aber indirekt durch vermehrte Proteinsynthese oder/und einen verminderten Proteinkatabolismus in den Myofibrillen zu einer Erhöhung der Muskelmasse (Int J Sports Med 21 (2000), 139-145). Als Ergebnis erhält man eine erhöhte fettfreie Körpermasse.

Neben dem Kreatin selbst, also insbesondere dem Kreatin Monohydrat, haben sich zwischenzeitlich auch zahlreiche Kreatinsalze, wie z.B. Kreatinascorbat, -citrat, -pyruvat und andere, ebenfalls als geeignete . Nahrungsergänzungsmittel erwiesen. Stellvertretend seien an dieser Stelle das europäische Patent EP 894 083 und die deutsche Offenlegungsschrift DE 197 07 694 A1 als Stand der Technik genannt.

Die für den Menschen als positiv nachgewiesenen Wirkungen entfaltet Kreatin auch bei Tieren, weshalb seine Verwendung in diversen Futtermitteln ebenfalls hinlänglich beschrieben ist. So ist in der internationalen Patentanmeldung WO 00/67 590 die Verwendung von Kreatin oder von Kreatin-Salzen als Futterzusatz für Zucht- und Masttiere, als Ersatz für Fleischmehl, Fischmehl und/oder antimikrobielle Leistungsförderer, Wachstumshormone sowie Anabolika beschrieben. GB 2 300 103 lehrt die Verwendung von Kreatin in Form eines Hundebiskuits, wofür das Kreatin Monohydrat gemeinsam mit Fleisch in einer extrudierten Masse angeboten wird. Da Kreatin Monohydrat aufgrund seiner schlechten Löslichkeit aber nur unzureichend bioverfügbar ist, wird seine gemeinsame Verwendung mit weiteren physiologisch aktiven Verbindungen, vorzugsweise in Salzform, empfohlen. Die deutsche Offenlegungsschrift DE 198 36 450 A1 hat die Verwendung stabiler Brenztraubensäure-Salze und insbesondere des Kreatinpyruvats in Formulierungen zum Gegenstand, die für die Tierernährung geeignet sind.

DE 100 03 835 A1 hat Formulierungen bei Dehydratationszuständen zum Gegenstand, wie sie generell bei älteren Personen und insbesondere solchen mit eingeschränkter Mobilität auftreten. In diesem Falle fungiert Kreatin als Transportmedium für Wasser, um den durch Dehydratationserscheinungen am stärksten betroffenen Geweben Feuchtigkeit zuzuführen.

Neben seinen unbestrittenen positiven physiologischen Eigenschaften besitzt Kreatin aber auch den Nachteil, dass es als Kreatin Monohydrat in wässrigen Lösungen keine ausgeprägte Stabilität besitzt, wobei es in Kreatinin umwandelt wird. Die Abbaugeschwindigkeit ist vom pH-Wert der Lösung und der Temperatur abhängig, wobei die Konzentration keine Rolle spielt. Besonders im sauren pH-Bereich verläuft dieser Abbau zu Kreatinin sehr schnell. Bei Raumtemperatur und pH 3,5 wird Kreatin bereits nach 3 Tagen zu über 20% in Kreatinin umgewandelt und geht somit für die physiologische Wirkung verloren. Ein pH von 3,5 stellt z.B. für ein Erfrischungsgetränk einen typischen pH-Wert dar. Aufgrund des schnellen Abbaus von Kreatin in diesem Milieu ist der Einsatz von Kreatin, insbesondere von Kreatin-Monohydrat, in wässrigen oder feuchten Formulierungen für die menschliche und tierische Ernährung praktisch ausgeschlossen. Bereits der pH-Wert im Magen von 1 bis 2 kann, je nach Verweilzeit, zu einem deutlichen Abbau des Kreatins zu Kreatinin führen. So konnte beim Menschen gezeigt werden, dass nach einer oralen Applikation von Kreatin nur etwa 15 bis 30 % des Kreatins von der Muskulatur resorbiert werden können (Greenhaff, P.L.: Factors Modifying Creatine Accumulation in Human Skeletal Muscle. In: Creatine. From Basic Science to Clinical Application. Medical Science Symposia Series Volume 14, 2000, 75-82).

Mehrere Arbeitsgruppen konnten bereits in den 50iger Jahren des letzten Jahrhunderts in klinischen Studien zeigen, dass die Verabreichung von Guanidinoessigsäure in Kombination mit Betain bei Herzkrankheiten einen positiven Einfluss auf den Krankheitsverlauf hat. Die Patienten berichteten von einer deutlichen Verbesserung ihres Allgemeinbefindens. Weiterhin wurde eine verbesserte Ausdauer bei körperlicher Belastung und eine erhöhte Muskelkraft schon nach kurzer Behandlungsdauer festgestellt. Auch berichteten die Patienten von einer verbesserten Libido. 200 Patienten wurde eine Dosis von 30mg/kg täglich über ein Jahr verabreicht. Nebenwirkungen konnten nicht beobachtet werden (Borsook H.; Borsook M.E.: The biochemical basis of betaine-glycocyamine therapy. In: Annals of western medicine and surgery 5(10), 825, 1951).

Es ist weiterhin bekannt, dass supplementierte Guanidinoessigsäure im Körper in Kreatin umgewandelt wird. So beschreibt WO 91/07954 die Verwendung von Guanidinoessigsäure bei physiologischen Zuständen, welche eine Erhöhung des Kreatinspiegels erfordern.

Im Zusammenhang mit der Überdosierung von Methionin ist ebenfalls bekannt, dass damit verbundene negative Effekte durch die Gabe von Guanidinoessigsäure abgemildert werden können (Interrelations of choline and methionine in growth and the action of betaine in replacing them.

McKittrick, D. S. Univ. of California, Berkeley, Archives of Biochemistry (1947), 15 133-55).

Die internationale Patentanmeldung WO 2004/000297 beschreibt eine Mischung, für die Ernährung oder für pharmazeutische Zwecke, welche für Säugetieren eingesetzt wird. Diese besteht aus einer Proteinfraktion welche L-Serin enthält und als weitere Komponente Guanidinoessigsäure. Die Mischung soll hierbei frei von Glycin sein oder nach der Hydrolyse der Mischung ein Verhältnis von L-Serin zu Glycin von größer als 2,7 zu 1 enthalten. Als mögliche Produktform werden Lösungen, Emulsionen, Suspensionen, Gele, Riegel, Süßigkeiten und vorzugsweise Pulver vorbeschrieben.

Von Guanidinoessigsäure ist weiterhin bekannt, dass sie eine antibakterielle Wirkung besitzt und in Tierversuchen erfolgreich gegen bakterielle Infektionen (Staphyllococcus aureus) eingesetzt werden konnte (Preparation for protecting mammals against infection (Stanley Drug Products Inc., USA). Neth. Appl. (1976), 7 pp. NL 7411216).

In jüngster Zeit wurde Guanidinoessigsäure auch als Nahrungsergänzungsmittel und Futtermittel eingesetzt. So konnte erst kürzlich gezeigt werden, dass Guanidinoessigsäure im Vergleich zu Kreatin eine wesentlich bessere Bioverfügbarkeit besitzt. In einem Fütterungsexperiment mit Hühnern wurde bereits bei Zugabe von weniger als 0,1 % Guanidinoessigsäure im Futter ein Gewichtszuwachs von 7 % und ein geringerer Futterverbrauch von 6 % gegenüber der Kontrollgruppe beobachtet. Im Gegensatz hierzu führte die Zugabe von 0,2 % Kreatin zum Futter nur zu einem Gewichtszuwachs von 4 % und einem geringeren Futterverbrauch von 2 bis 3 %.

Weiterhin konnte gezeigt werden, dass Guanidinoessigsäure seine maximale Wirkung bereits bei einer Dosierung entfaltet, bei der Kreatin zu keinem beobachtbaren Effekt führt. Der verbesserte Gewichtszuwachs und die bessere Futterverwertung bei sehr niedriger Dosierung ist mit einer hohen Umwandlungsrate der aufgenommenen Guanidinoessigsäure in Kreatin zu erklären. So führte bereits eine Zugabe von 0,032 % Guanidinoessigsäure zum Hühnerfutter zu einem Gewichtszuwachs von 3% und einer verbesserten Futterverwertung von 3% (WO 2005/120246 A1). Dies deckt sich auch mit der Beobachtung, dass das Enzym Transmethylase in sehr hohen Konzentrationen in der Leber gefunden wird.

Wegen der verhältnismäßig schlechten Löslichkeit von Guanidinoessigsäure in Wasser wurde bereits versucht die Löslichkeit zu verbessern und die Bioverfügbarkeit weiter zu erhöhen, wobei gleichzeitig die bekannt guten physiologischen Eigenschaften der Guanidinoessigsäure erhalten bleiben sollten. Zu diesem Zweck wurden neue, stabile Salze und/oder Anlagerungs-und/oder Komplexverbindungen der Guanidinoessigsäure mit Äpfelsäure, Asparaginsäure, Ascorbinsäure, Bernsteinsäure, Brenztraubensäure, Fumarsäure, Gluconsäure, α-Ketoglutarsäure, Oxalsäure, Pyroglutaminsäure, 3-Nicotinsäure, Milchsäure, Zitronensäure, Maleinsäure, Schwefelsäure, Essigsäure, Ameisensäure, 2-Hydroxybenzoesäure, L-Carnitin, Acetyl-L-Carnitin, Taurin, Betain, Cholin, Methionin und Liponsäure sowie als Natrium-, Kalium- oder Calciumguanidinoacetat bereitgestellt (DE 10 2005 009 990.4; noch unveröffentlicht).

Mit diesen neuen Verbindungen konnte gegenüber der freien Guanidinoessigsäure eine höhere Wasserlöslichkeit erreicht werden und auch hinsichtlich ihrer Stabilität und Bioverfügbarkeit sind diese Verbindungen der freien Guanidinoessigsäure mindestens ebenbürtig.

US 2 761 807 A offenbart eine therapeutische Zusammensetzung, welche Guanidinessigsäure (Glycocyamine) und einen Methyldonor aus der Reihe Betain, Betainhydrat, Cholin oder Dimethylthetin enthält, zur Behandlung von Muskel- und Nervenbeschwerden. Eine bevorzugte Darreichungsform ist eine Suspension von Guanidinessigsäure (d.h. eine Guanidinessigsäure-Komponente) in einer wäßrigen Lösung des Methyldonors. Die hinzugefügte Wassermenge sollte ausreichen um die Einnahme der Zusammensetzung durch die Patienten zu erleichtern.

WO 2004/000042 A offenbart nutritionelle oder pharmazeutische Zusammensetzungen, welche Methyldonoren, z.B. Methionin, Cholin, Betain, und Methylakzeptoren, z.B. Guanidinessigsäure enthalten, zur Vorbeugung oder Behandlung katabolischer Prozesse. In einer bevorzugten Ausführungsform ist die Zusammensetzung eine Lösung, welche min. 80% Wasser enthält.

Aus den bzgl. Kreatin geschilderten Nachteilen des Standes der Technik hat sich für die vorliegende Erfindung die Aufgabe gestellt, wässrige Formulierungen für die menschliche Ernährung zu finden, welche nach Möglichkeit eine geringe Instabilität bei industriellen Verarbeitungsprozessen besitzen. Weiterhin sollten sie hohe Verarbeitungstemperaturen, wie sie bei der Sterilisierung auftreten, unbeschadet überstehen und auch in industriell hergestellten Fertiggetränken über Monate lagerstabil sein. Weiterhin sollte die Verbindung im Gegensatz zu Kreatin das saure Milieu des Magens unbeschadet überstehen und erst nach der Aufnahme in den Körper in Kreatin umgewandelt werden. Die eingesetzte Formulierung sollte selbst keine physiologisch nachteiligen Wirkungen entfalten und leicht nachweisbar sein. Unter wirtschaftlichen Gesichtspunkten stand für die erfindungsgemäß zu verwendenden Substanzen mit im Vordergrund, diese auf wirtschaftlich günstige Weise herzustellen.

Gelöst wurde diese Aufgabe durch die Bereitstellung einer Flüssigformulierung, bestehend aus einer wässrigen Lösung mindestens einer Guanidinoessigsäure-Komponente und einem Methylgruppen-Donor aus der Reihe Cholin, Methionin und Betain, wie beansprucht.

Überraschend hat sich herausgestellt, dass mit dieser Formulierung nicht nur die Aufgabenstellung voll erfüllt werden konnte, indem die darin enthaltenen Guanidinoessigsäure-Komponenten auch über längere Zeit in diesen wasserhaltigen Zubereitungen stabil sind und im Körper sehr schnell in Kreatin umgewandelt werden. Im Herstellungsprozess müssen wässrige Zubereitungen, wie sie auch solche gemäß Erfindung darstellen, in der Regel pasteurisiert oder sterilisiert werden. Hierbei wurde überraschend gefunden, dass Guanidinoessigsäure im Gegensatz zu Kreatin, auch unter diesen zum Teil extremen Bedingungen, eine herausragende Stabilität besitzt. Diese Vorteile waren in ihrer Gesamtheit so nicht zu erwarten.

Als Guanidinoessigsäure-Komponente sieht die vorliegende Erfindung Guanidinoessigsäure und/oder mindestens ein Salz, eine Anlagerungs- oder Komplexverbindung davon vor.

Besonders bevorzugt sollte es sich erfindungsgemäß bei der Guanidinoessigsäure-Komponente um Verbindungen zwischen Guanidinoessigsäure und Natrium, Kalium oder Calcium handeln.

Das Mengenverhältnis von Guanidinoessigsäure-Komponente zum Methylgruppen-Donor kann in weiten Grenzen variiert werden. Es hat sich jedoch als besonders vorteilhaft erwiesen, die Guanidinoessigsäure-Komponente und der Methylgruppen-Donor in einem Gewichtsverhältnis von 1 : 10 bis 10 : 1 einzusetzen.

Besonders bevorzugt weist die erfindungsgemäße Flüssig-Formulierung einen Wassergehalt ≥ 10 Gew.-%, insbesondere ≥ 20 Gew.-%, bezogen auf das Gesamtgewicht, auf.

Selbstverständlich ist die vorgeschlagene Formulierung nicht auf die Guanidinoessigsäure-Komponente als alleinigen Wirkstoff beschränkt. Aus diesem Grund sieht die vorliegende Erfindung auch eine Variante vor, bei der die Formulierung weitere physiologisch aktive Verbindungen enthalten kann, die aus der Reihe Kohlenhydrate, Fette, Aminosäuren, Proteine, Vitamine, Mineralstoffe, Spurenelemente sowie deren Derivaten und Mischungen ausgewählt werden.

Im Vergleich zu Kreatin besitzt Guanidinoessigsäure eine geringere Löslichkeit in Wasser (3,8 g pro Liter bei Raumtemperatur). Dies ist jedoch für die beanspruchte Zubereitung nicht von Nachteil, da Guanidinoessigsäure bereits in einem deutlich niedrigeren Dosierbereich als Kreatin Monohydrat seine Wirkung entfaltet. Während für Kreatin Monohydrat Tagesdosen von 5 bis 20 g üblich sind, konnten bei Verabreichung einer Tagesdosis von 2 g Guanidinoessigsäure bereits ausgeprägt positive Effekte beobachtet werden (Borsook H.; Borsook M.E.: The biochemical basis of betaine-glycocyamine therapy. In: Annals of western medicine and surgery 5(10), 825, 1951). Somit kann z.B. bereits in einem halben Liter eines wässrigen Getränks problemlos eine physiologisch sinnvolle Tagesdosis der Guanidinoessigsäure-Komponente eingearbeitet werden. Aufgrund des in letzter Zeit zunehmenden Angebots an geeigneten Guanidinoessigsäuresalzen, sind aber auch Lösungen mit deutlich höheren Konzentrationen der Guanidinoessigsäure-Komponente möglich.

Aufgrund der unerwarteten positiven Eigenschaften berücksichtigt die vorliegende Erfindung als weitere Variante die Möglichkeit, dass die Zubereitung als Mineralwasser, Limonade, Sport-, Mineral-, Frucht-, Fruchtsaft-, Milch-, Molke- oder alkoholhaltiges Getränk oder als Trinkwasserzubereitung vorliegt.

Die Formulierung ist bzgl. der Guanidinoessigsäure-Komponente nicht limitiert, wobei insbesondere die Mengen der Guanidinoessigsäure-Komponente, in denen sie in der Zubereitung vorliegen kann, keine Einschränkung darstellt. Aus wirtschaftlichen und ernährungsphysiologischen Gründen werden allerdings Mengen empfohlen, die zwischen 0,01 und 4 Gew.-% liegen. Besonders bevorzugt sind Mengen zwischen 2,5 und 4,0 Gew.-% und insbesondere 3,8 Gew.-%.

Die vorliegende Erfindung berücksichtigt auch die Verwendung der beanspruchten Zubereitung als physiologisches Stärkungsmittel und in diesem Zusammenhang insbesondere in Form eines Funktionsnahrungsmittels (Functional Food) für Menschen, wobei der Schul-, Sport-, Rekonvaleszenz- und/oder Geriatrie-Bereich im Vordergrund stehen.

Selbstverständlich ist es auch möglich, die vorgeschlagene Zubereitung gemeinsam mit Nahrungsergänzungsmitteln zu verwenden, was die vorliegende Erfindung ebenfalls vorsieht. Hier ist insbesondere der medizinische Bereich von Interesse.

Insgesamt stellt die vorgeschlagene Formulierung, deren wässrige Lösung einen bevorzugten pH-Bereich zwischen 2,5 und 11 besitzt, und ihre Verwendung eine weitere Fortentwicklung des Standes der Technik hinsichtlich der freien Guanidinoessigsäure und ihrer Salze und Anlagerungsverbindungen in Kombination mit einen Methylgruppendonor aus der Reihe Cholin, Methionin und Betain dar. Es ist nun nämlich möglich, diese Verbindungen nicht nur in trockenen Zubereitungen einzusetzen, sondern auch als lagerstabile Lösungen, wobei sich die vorgeschlagenen Formulierungen auch ausgezeichnet für die industrielle Herstellung von Getränken eignen. Guanidinoessigsäure und deren Salze, sowie Anlagerungs- oder Komplexverbindung sind auch über mehrere Monate in den neuen Formulierungen stabil und sie können darüber hinaus dem Körper in ausgezeichneter Bioverfügbarkeit zugeführt werden, wobei die jeweils verabreichte Guanidinoessigsäure-Komponente im Körper sehr schnell in Kreatin umgewandelt wird.

Die nachfolgenden Beispiele veranschaulichen die Vorteile der vorliegenden Erfindung.

### Beispiele

### 1. Nahrungsergänzungsmittel

Nachfolgend sind typische Zusammensetzungen von wohlschmeckenden Formulierungen aufgeführt, deren Bestandteile bei Raumtemperatur in 500 ml Fruchtsaft und/oder Wasser und/oder Joghurt und/oder Molke eingebracht werden.

| | | |
|---|---|---|
| 1.1 | 1.500 mg | Glucosamin |
| | 1.800 mg | Guanidinoessigsäure |
| | 3.600 mg | Betain |
| | 720 mg | Magnesium-L-hydrogenaspartat |
| | 2.000 mg | Glucose |
| | 500 mg | Ascorbinsäure |
| | | |
| 1.2 | 400 mg | Chondroitinsulfat |
| | 4.000 mg | Guanidinoessigsäure-citrat |
| | 8.000 mg | Betain |
| | 2.000 mg | Dicalciumphosphat |
| | 400 mg | (MgCO₃)₄·Mg(OH)₂·5H₂O = ca. 100 Mg |
| | 500 mg | Vitamin C |
| | | |
| 1.3 | 1.000 mg | Glucosamin |
| | 300 mg | Chondroitinsulfat |
| | 2.800 mg | Guanidinoessigsäure-pyruvat |
| | 6.000 mg | Betain |
| | 500 mg | Methionin |
| | 3.100 mg | Creatinol-phosphat |

### 2. Lagerstabilität:

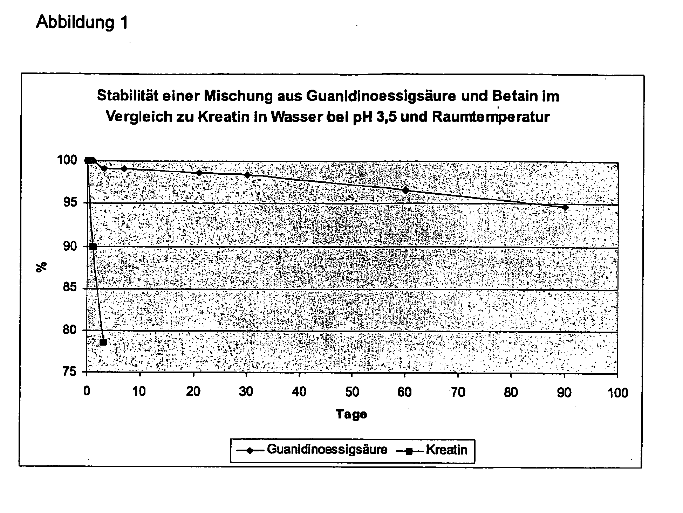

Gemäß Abbildung 1 wurde die Lagerstabilität von Kreatin im Vergleich zu einer Mischung aus 4 Gew.-Teilen Guanidinoessigsäure und 6 Gew.-Teilen Betain in wässriger Lösung bei pH 3,5 und Raumtemperatur bestimmt:
Während sich Kreatin nach 3 Tagen bereits zu über 20 % in Kreatinin umsetzt, waren bei der Mischung aus Guanidinoessigsäure und Betain unter identischen Bedingungen nach 90 Tagen noch 95 % der eingesetzten Menge an Guanidinoessigsäure nachweisbar. Betain ist unter den angegebenen Bedingungen vollkommen stabil.

## Patentansprüche

1. Flüssig-Formulierung bestehend aus einer wässrigen Lösung einer Guanidinoessigsäure-Komponente und mindestens einem Methylgruppen-Donor der Reihe Cholin, Methionin und Betain zur Herstellung eines industriell hergestellten Fertiggetränks, **dadurch gekennzeichnet, dass** die Flüssig-Formulierung die Guanidinoessigsäure-Komponente in Mengen von 0,1 bis 4,0 g/l enthält und wobei es sich bei der Guanidinoessigsäure-Komponente um Guanidinoessigsäure und/oder mindestens ein Salz, eine Anlagerungs- oder Komplexverbindung davon handelt.

2. Nicht-therapeutische Verwendung einer Flüssig-Formulierung bestehend aus einer wässrigen Lösung einer Guanidinoessigsäure-Komponente und mindestens einem Methylgruppen-Donor der Reihe Cholin, Methionin und Betain in einem industriell hergestellten Fertiggetränk, **dadurch gekennzeichnet, dass** die Flüssig-Formulierung die Guanidinoessigsäure-Komponente in Mengen von 0,1 bis 4,0 g/l enthält und wobei es sich bei der Guanidinoessigsäure-Komponente um Guanidinoessigsäure und/oder mindestens ein Salz, eine Anlagerungs- oder Komplexverbindung davon handelt.

3. Formulierung oder Verwendung nach einem der Ansprüche 1 bis 2, **dadurch gekennzeichnet, dass** es sich bei der Guanidinoessigsäure-Komponente um Verbindungen zwischen Guanidinoessigsäure und Natrium, Kalium oder Calcium handelt.

4. Formulierung oder Verwendung nach einem der Ansprüche 1 bis 3, **dadurch gekennzeichnet, dass** die Guanidinoessigsäure-Komponente und der Methylgruppen-Donor in einem Gew.-Verhältnis von 1 : 10 bis 10 : 1 eingesetzt werden.

5. Formulierung oder Verwendung nach einem der Ansprüche 1 bis 4, **dadurch gekennzeichnet, dass** die Flüssig-Formulierung weitere physiologisch aktive Verbindungen der Reihe Kohlenhydrate, Fette, Aminosäuren, Proteine, Vitamine, Mineralstoffe, Spurenelemente sowie deren Derivate und Mischungen daraus enthält.

6. Formulierung oder Verwendung nach einem der Ansprüche 1 bis 5 in Form von Mineralwasser, Limonade, Sport-, Mineral-, Frucht-, Fruchtsaft-, Milch-, Molke- oder alkoholhaltigem Getränk oder als Trinkwasserzubereitung.

7. Formulierung oder Verwendung nach einem der Ansprüche 1 bis 6 als physiologisches Stärkungsmittel und als Funktionsnahrungsmittel (Functional Food) für Menschen.

8. Formulierung oder Verwendung nach Anspruch 7 im Schul-, Sport-, Rekonvaleszenz- und/oder Geriatrie-Bereich.

9. Formulierung oder Verwendung nach Anspruch 8, **dadurch gekennzeichnet, dass** die Formulierung gemeinsam mit Nahrungsergänzungsmitteln eingesetzt wird.

10. Formulierung nach Anspruch 9, **dadurch gekennzeichnet, dass** die Formulierung im medizinischen Bereich eingesetzt wird.

11. Formulierung oder Verwendung nach einem der Ansprüche 1 bis 10, **dadurch gekennzeichnet, dass** die wässrige Lösung einen pH zwischen 2,5 und 11 besitzt.

12. Industriell hergestelltes Fertiggetränk umfassend eine Flüssig-Formulierung bestehend aus einer wässrigen Lösung aus einer Guanidinoessigsäure-Komponente und mindestens einem Methylgruppen-Donor der Reihe Cholin, Methionin und Betain, **dadurch gekennzeichnet, dass** die Flüssig-Formulierung die Guanidinoessigsäure-Komponente in Mengen von 0,1 bis 4,0 g/l enthält und wobei es sich bei der Guanidinoessigsäure-Komponente um Guanidinoessigsäure und/oder mindestens ein Salz, eine Anlagerungs- oder Komplexverbindung davon handelt.

## Claims

1. Liquid formulation consisting of an aqueous solution of a guanidinoacetic acid component and at least one methyl group donor selected from the series choline, methionine, and betaine for producing an industrially produced ready-to-drink product, **characterised in that** the liquid formulation contains the guanidinoacetic acid component in amounts of from 0.1 to 4.0 g/l and the guanidinoacetic acid component being guanidinoacetic acid and/or at least one salt, addition compound or complex compound thereof.

2. Non-therapeutic use of a liquid formulation consisting of an aqueous solution of a guanidinoacetic acid component and at least one methyl group donor from the series choline, methionine, and betaine in an industrially produced ready-to-drink product, **characterised in that** the liquid formulation contains the guanidinoacetic acid component in amounts of from 0.1 to 4.0 g/I and the guanidinoacetic acid component being guanidinoacetic acid and/or at least one salt, addition compound or complex compound thereof.

3. Formulation or use according to either claim 1 or claim 2, **characterised in that** the guanidinoacetic acid component is compounds between guanidinoacetic acid and sodium, potassium or calcium.

4. Formulation or use according to any of claims 1 to 3, **characterised in that** the guanidinoacetic acid compound and the methyl group donor are used in a weight ratio of from 1:10 to 10:1.

5. Formulation or use according to any of claims 1 to 4, **characterised in that** the liquid formulation contains further physiologically active compounds from the series carbohydrates, fats, amino acids, proteins, vitamins, minerals, trace elements and derivatives and mixtures thereof.

6. Formulation or use according to any of claims 1 to 5 in the form of mineral water, lemonade, a sports drink, a mineral drink, a fruit drink, a fruit juice drink, a milk drink, a whey drink, an alcoholic beverage or as a drinking water preparation.

7. Formulation or use according to any of claims 1 to 6 as a physiological tonic and as functional food for humans.

8. Formulation or use according to claim 7 in the school, sport, convalescence and/or geriatric sectors.

9. Formulation or use according to claim 8, **characterised in that** the formulation is used together with food supplements.

10. Formulation according to claim 9, **characterised in that** the formulation is used in the medical sector.

11. Formulation or use according to any of claims 1 to 10, **characterised in that** the aqueous solution has a pH of between 2.5 and 11.

12. Industrially produced ready-to-drink product comprising a liquid formulation consisting of an aqueous solution of a guanidinoacetic acid component and at least one methyl group donor from the series choline, methionine, and betaine, **characterised in that** the liquid formulation contains the guanidinoacetic acid component in amounts of from 0.1 to 4.0 g/I and the guanidinoacetic acid component being guanidinoacetic acid and/or at least one salt, addition compound or complex compound thereof.

## Revendications

1. Formulation liquide consistant en une solution aqueuse d'un composant acide guanidinoacétique et d'au moins un donneur de radical méthyle de la série de la choline, la méthionine et la bétaïne pour préparer une boisson prête à boire, préparée industriellement, **caractérisée en ce que** la formulation liquide contient le composant acide guanidinoacétique en une quantité allant de 0,1 à 4,0 g/litre et **en ce que** le composant acide guanidinoacétique consiste en l'acide guanidinoacétique et/ou au moins un sel, un composé d'addition ou de complexation de celui-ci.

2. Utilisation non thérapeutique d'une formulation liquide consistant en une solution aqueuse d'un composant acide guanidinoacétique et d'au moins un donneur de radical méthyle de la série de la choline, la méthionine et la bétaïne dans une boisson prête à boire, préparée industriellement, **caractérisée en ce que** la formulation liquide contient le composant acide guanidinoacétique en une quantité allant de 0,1 à 4,0 g/litre et **en ce que** le composant acide guanidinoacétique consiste en l'acide guanidinoacétique et/ou au moins un sel, un composé d'addition ou de complexation de celui-ci.

3. Formulation ou utilisation selon l'une des revendications 1 à 2, **caractérisée en ce que** le composant acide guanidinoacétique consiste en des composés de l'acide guanidinoacétique et du sodium, du potassium ou du calcium.

4. Formulation ou utilisation selon l'une des revendications 1 à 3, **caractérisée en ce que** le composant acide guanidinoacétique et le donneur de radical méthyle sont mis en oeuvre en un rapport pondéral allant de 1:10 à 10:1.

5. Formulation ou utilisation selon l'une des revendications 1 à 4, **caractérisée en ce que** la formulation liquide contient d'autres composés physiologiquement actifs, choisis parmi des hydrates de carbone, des graisses, des acides aminés, des protéines, des vitamines, des minéraux, des oligoéléments ainsi que leurs dérivés et mélanges.

6. Formulation ou utilisation selon l'une des revendications 1 à 5, sous forme d'eau minérale, de limonade, de boisson pour le sport, minérale, aux fruits, de type jus de fruit, de type lait, de type petit-lait ou alcoolisée ou comme composition d'eau potable.

7. Formulation ou utilisation selon l'une des revendications 1 à 6, comme soutien physiologique et comme aliment fonctionnel (Functional Food) pour l'homme.

8. Formulation ou utilisation selon la revendication 7, dans le domaine scolaire, du sport, de la convalescence et/ou de la gériatrie.

9. Formulation ou utilisation selon la revendication 8, **caractérisée en ce que** la formulation est mise en oeuvre avec un complément alimentaire.

10. Formulation selon la revendication 9, **caractérisée en ce que** la formulation est mise en oeuvre dans le domaine médical.

11. Formulation ou utilisation selon l'une des revendications 1 à 10, **caractérisée en ce que** la solution aqueuse possède un pH allant de 2,5 à 11.

12. Boisson prête à boire, préparée industriellement, comprenant une formulation liquide consistant en une solution aqueuse d'un composant acide guanidinoacétique et d'au moins un donneur de radical méthyle de la série de la choline, la méthionine et la bétaïne, **caractérisée en ce que** la formulation liquide contient le composant acide guanidinoacétique en une quantité allant de 0,1 à 4,0 g/litre et **en ce que** le composant acide guanidinoacétique consiste en l'acide guanidinoacétique et/ou au moins un sel, un composé d'addition ou de complexation de celui-ci.
